# EUROPEAN PATENT SPECIFICATION

(11) **EP 2 353 627 B1**
(45) Date of publication and mention of the grant of the patent: **14.06.2017**
(21) Application number: 11152878.2
(22) Date of filing: 01.02.2011
(51) Int. Cl.: A61M 5/142, F04B 43/08, F04B 43/12, A61M 5/14

(54) **Peristaltic fluid pump with detachable cassette**
Peristaltische Flüssigkeitspumpe mit modularer Kassette
Pompe péristaltique avec cassette

(30) Priority: 03.02.2010 JP 2010021882
(43) Date of publication of application: 10.08.2011
(73) Proprietor: Seiko Epson Corporation, Shinjuku-ku Tokyo (JP)
(72) Inventor: Miyazaki, Hajime, Suwa-shi Nagano 392-8502 (JP); Uchikawa, Daisuke, Suwa-shi Nagano 392-8502 (JP)
(74) Representative: Hoffmann Eitle

(56) References cited:
- EP-A1- 1 810 702
- EP-A1- 2 123 313
- EP-A2- 2 169 225
- US-A- 4 493 706
- US-A- 5 318 413
- US-A- 5 364 342
- US-A- 5 538 405
- US-A1- 2005 245 888
- US-A1- 2008 138 218
- US-A1- 2009 208 350

## Description

### BACKGROUND

### 1. Technical Field

The present invention relates to a technology which transports fluid by pressing a tube.

### 2. Related Art

A pump of a type which presses a tube (tube pump) is used as a device for transporting fluid. This tube pump chiefly includes a tube made of elastic material, and a pressing member which presses the tube to close at least one position of the tube. A known type of the pressing member has a rotor rotated by a driving unit such as a step motor, and a roller attached to the rotor to press the tube (for example, see Japanese Patent No. 3177742). When the roller shifts by rotation of the rotor, the closed position of the tube moves accordingly. As a result, fluid within the tube is pushed toward the downstream side, and fluid on the upstream side is sucked into the tube by the restoring force of the tube after the roller passes. This tube pump achieves stable transportation at low speed, and thus is used in the medical field which requires high accuracy of transportation sufficient for giving a specified amount of a liquid medicine, for example.

According to this type of tube pump so constructed as to be kept pressed and closed at least at one position for allowing push and suck of fluid in accordance with the movement of the closed position of the tube, however, the pressed position of the tube deteriorates and weakens the restoring force of the tube when the period from the manufacture of the tube pump to the time of the practical use is long. In this case, the accuracy of fluid transportation lowers.

Moreover, when a liquid medicine is transported as fluid by using this tube pump, the liquid medicine which easily evaporates from the tube gradually decreases during transportation. In this case, a specified amount of the liquid medicine is difficult to be transported with high accuracy. Particularly, when a small amount of a liquid medicine is slowly transported through a narrow tube of a miniaturized tube pump, the amount of evaporation relative to the amount of transportation of the liquid medicine increases and thus makes the problem more remarkable and serious.

As a further prior art document, EP 2 123 313 A1 refers to a method of forming a body fluid purification cassette according to the preamble of claim 1. Further, US 5,318,413 A1 refers to a peristaltic pump, which is provided with an adjustable flow regulation provided by an adjustable axial elongation means for the variable elongation of resilient tubing disposed between the associated motors of a rotary peristaltic pump or a plurality of reciprocal pushed or fingers in a linear peristaltic pump to provide a radial compression of successive sections of the resilient tubing. Another prior art document is US 4,493,706 A1, which refers to a linear peristaltic pump.

### SUMMARY

An advantage of some aspects of the invention is to provide a technology capable of preventing deterioration of a tube caused by continuous press of the tube and increasing accuracy of fluid transportation so as to solve the above problems arising from the technology in the related art.

According to an aspect of the invention, there is provided a fluid transporter according to claim 1.

According to the fluid transporter of this aspect of the invention, the attachment case containing the flow path is so constructed as to be attachable to and detachable from the main body case on which the pressing member for pressing the tube is provided. When the attachment case is attached to the main body case, the tube constituting the flow path is pressed by the pressing member and closed. The portion of the flow path pressed by the pressing member is formed by the tube, and the remaining portion of the flow path is formed integrally with the attachment case. The pressing member is not limited
to a component directly pressing the tube but may be a component indirectly pressing the tube via another part as long as the tube can be pressed by attachment of the attachment case to the main body case.

According to the fluid transporter of the aspect of the invention having this structure, the attachment case having the tube is detachably attached to the main body case having the pressing member. In this case, the tube is not constantly pressed, which avoids deterioration of the tube. Moreover, only the pressed portion of the flow path is formed by the tube, and the remaining portion is formed integrally with the attachment case. Thus, the accuracy of fluid transportation increases. It is known that vaporized fluid easily goes out from a tube made of elastic material. Therefore, when a liquid medicine is transported as fluid by using a fluid transporter, a specified amount of the liquid medicine is difficult to be accurately transported (given) due to evaporation of the liquid medicine from the tube during transportation. However, when the portion of the flow path other than the pressed portion is formed integrally with the attachment case, the portion of the flow path formed integrally with the attachment case has a higher gas-barrier property than that of the tube. Thus, decrease in the fluid during transportation can be reduced more than in the structure which forms the entire flow path by the tube. Accordingly, the accuracy of liquid medicine transportation by using the fluid transporter increases.

Moreover, the structure which forms the portion not pressed by the pressing member integrally with the attachment case contributes to the improvement of the accuracy of fluid transportation in the following viewpoints as well. Generally, the inside diameter of a tube formed by extrusion molding varies for each manufacturing lot, and this variation changes the flow amount of fluid flowing within the tube and thus greatly affects the accuracy of fluid transportation. However, in case of the portion of the flow path formed integrally with the attachment case and shaped by mold forming, the accuracy of dimensions increases. In this case, the accuracy of fluid transportation becomes higher than that of the structure which forms the entire flow path by the tube. When the tube constituting the flow path is short, the tube can be formed not by extrusion molding but by mold forming. In this case, the variation of the inside diameter of the tube decreases, and thus the accuracy of fluid transportation further improves.

In addition, when the portion not pressed by the pressing member is formed integrally with the attachment case, the size of the fluid transporter can be reduced. In case of a structure which draws the tube in a circular shape within the attachment case to form the entire flow path by the tube, a radius of curvature R needs to be large enough so as not to bend and close the tube. However, in case of a structure which forms the portion of the flow path pressed by the pressing member by the tube and forms the remaining portion integrally with attachment case as in the fluid transporter of this aspect of the invention, the radius of curvature R of the portion of the flow path formed integrally with the attachment case can be reduced (the portion of the flow path can be bended at an acute angle). Thus, the fluid transporter can be made compact.

Moreover, when only the portion of the flow path pressed by the pressing member is formed by the tube, the attachment error of the tube (such as expansion of the tube caused by pulling) is less likely to be produced than in the structure which forms the entire flow path by the tube. Thus, the fluid transporter can be easily manufactured.

Furthermore, when only the pressed portion of the flow path is formed by the tube, the amount of the expensive tube used for the manufacture of the fluid transporter decreases. Thus, the manufacturing cost of the fluid transporter lowers.

The fluid transporter of this aspect of the invention further includes a control unit which changes the inside diameter of the tube with the tube attached to the attachment case.

As explained above, the inside diameter of the tube varies for each manufacturing lot, and further variation of the inside diameter of the tube may be produced by pulling or press fitting of the tube at the time of attachment of the tube to the attachment case. The flow amount of the fluid flowing within the tube changes by these variations and thus greatly affects the accuracy of fluid transportation. When the control unit for changing the inside diameter of the tube is provided, the variation of the inside diameter of the tube can be corrected with the tube attached to the attachment case. Thus, the accuracy of fluid transportation increases.

According to the fluid transporter of this aspect of the invention, the inside diameter of the tube is changed by expanding and contracting the tube in accordance with rotation of a rotation mechanism.

According to this structure, when the inside diameter of the tube is larger than the standard diameter due to the variation in the manufacture, for example, the inside diameter of the tube can be decreased by extending the tube through the rotation of the rotation mechanism in the direction for increasing the length of the tube under the condition that the tube is attached to the attachment case. By this method, the variation of the inside diameter of the tube can be easily corrected for higher accuracy of fluid transportation.

According to the fluid transporter of this aspect of the invention, the pressing member may include a cam provided on the main body case so as to be along the side surface of the tube, and a plurality of pressing shafts provided on the attachment case to sequentially press the tube by rotation of the cam.

When a tube is directly pressed by a cam and closed, the cam rotated for shifting the closed position slides on the side surface of the tube and thus generates abrasion of the tube. However, in case of the structure which sequentially pushes the plural pressing shafts disposed along the side surface of the tube by using the cam, the respective pressing shafts only press the predetermined position of the tube. Thus, no abrasion of the tube is produced by the sliding, which increases the durability of the tube.

According to the fluid transporter of this aspect of the invention, the proportion of the length of the tube to the entire length of the flow path may be set at half or shorter than half the entire length of the flow path.

In this structure, the evaporation of the liquid medicine from the tube, and the effect of the variation of the inside diameter in the manufacture of the tube imposed on the accuracy of fluid transportation as described above can be reduced to half. This structure is desirable because the accuracy of fluid transportation achieved by the fluid transporter increases by the reduction of the effect of the variation. Moreover, in the structure which has only the short tube for forming the flow path and constitutes most part of the flow path by the portion formed integrally with the attachment case, the radius of curvature R for bending can be decreased as explained above. Accordingly, the degree of freedom for the layout of the flow path increases, and thus reduction of the size of the fluid transporter can be easily achieved.

According to the fluid transporter of this aspect of the invention, the inside diameter of the portion of the flow path provided on the attachment case may be made smaller than the inside diameter of the tube.

At the time of the manufacture of the fluid transporter, the flow path is not filled with fluid as the target of transportation in most cases. Thus, for initiating the use of the fluid transporter, initial filling for filling the flow path with fluid as the target of transportation (initial filling) needs to be completed in the first place. The flow amount of the fluid flowing within the flow path is chiefly determined by the inside diameter of the tube pressed by the pressing member and the driving speed of the pressing member. Also, the flow speed of the fluid in the path provided on the attachment case increases as the inside diameter of this path decreases. Thus, when the inside diameter of the path provided on the attachment case is shorter than the inside diameter of the tube, the time required for the initial filling can be reduced while maintaining the flow amount of the fluid flowing within the flow path. In addition, reduction of the inside diameter of the path provided on the attachment case is less likely to increase the variation of the inside diameter of the path produced in the manufacture than in case of reduction of the inside diameter of the tube.

### BRIEF DESCRIPTION OF THE DRAWINGS

The invention will be described with reference to the accompanying drawings, wherein like numbers reference like elements.
Fig. 1 illustrates the general structure of a tube pump as a fluid transporter according to an embodiment.
Figs. 2A and 2B illustrate the structures of a main body of the tube pump and a cartridge, respectively.
Fig. 3 illustrates a condition in which the cartridge is attached to the main body of the tube pump according to the embodiment.
Figs. 4A and 4B are cross-sectional views each of which shows a condition in which a tube is pressed by a pressing shaft.
Fig. 5 is a cross-sectional view showing the structure of an upstream path provided on the cartridge, and the connection between the upstream path and the tube according to the embodiment.
Fig. 6 is a cross-sectional view showing the structure of a reservoir included in the cartridge according to the embodiment.

### DESCRIPTION OF EXEMPLARY EMBODIMENT

For clarifying the details of the invention, an embodiment of the invention is hereinafter described in the following order.
A. Device Structure:
B. Fluid Transportation Operation in This Embodiment:

### A. Device Structure

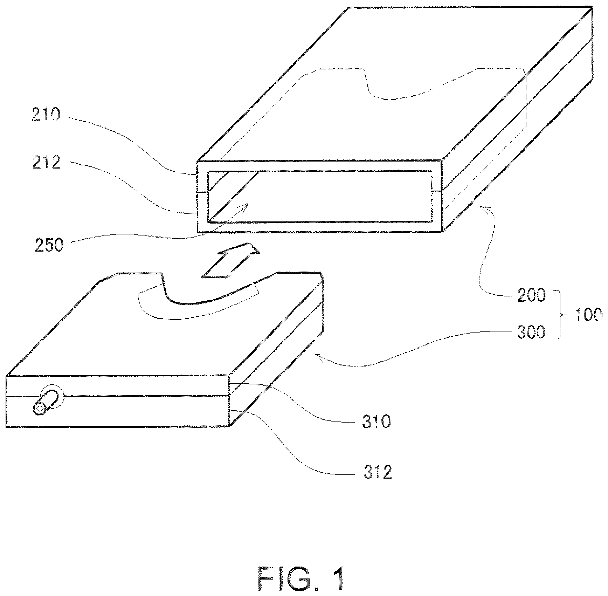

Fig. 1 illustrates the general structure of a tube pump as a fluid transporter according to this embodiment. As illustrated in the figure, a tube pump 100 in this embodiment chiefly includes a main body 200, and a cartridge 300 attachable to and detachable from the main body 200. The main body 200 is formed by a first main body case 210 and a second main body case 212 affixed to each other, and the cartridge 300 is formed by a first cartridge case 310 and a second cartridge case 312 affixed to each other. Each of the first main body case 210, the second main body case 212, the first cartridge case 310, and the second cartridge case 312 is made of lightweight material having a certain strength such as plastics, and more preferably made of material having suitability for a living body.

The main body 200 further includes an attachment space 250 to which the cartridge 300 is attached. The tube pump 100 functions as a fluid transporter with the cartridge 300 attached to the attachment space 250. In Fig. 1, the attachment space 250 contained in the main body 200 is indicated by a broken line. The respective structures of the main body 200 and the cartridge 300 are now explained in more detail.

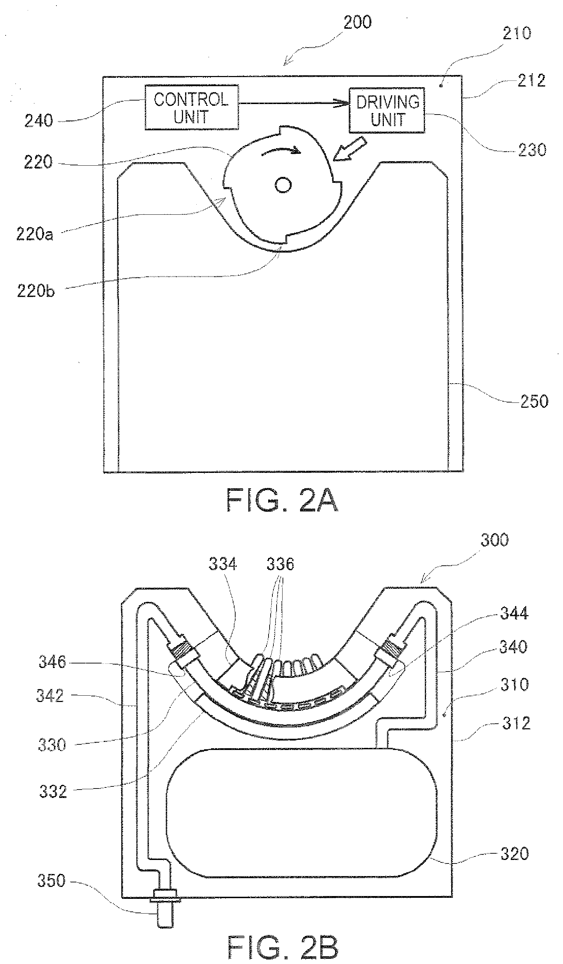

Figs. 2A and 2B illustrate the structures of the main body 200 of the tube pump 100 and the cartridge 300, respectively. Fig. 2A shows the structure of the main body 200, and Fig. 2B shows the structure of the cartridge 300. As illustrated in Fig. 2A, a cam 220, a driving unit 230 for rotating the cam 220, a control unit 240 for controlling the operation of the driving unit 230 and others are mounted on the main body 200. As explained above, the external frame of the main body 200 is constituted by the first main body case 210 and the second main body case 212, and the components of the cam 220, the driving unit 230, and the control unit 240 are disposed inside the external frame. Fig. 2A shows the structure assuming that the first main body case 210 is made of transparent material. In addition, the main body 200 has the attachment space 250 to which the cartridge 300 is attached as described above.

The driving unit 230 in this embodiment has a so-called step motor to rotate the cam 220 clockwise by driving the step motor. The driver included in the driving unit 230 is not limited to the step motor but may be other types of driver as long as they can give a rotational force to the cam 220.

Concave portions 220a each of which has the smallest radius from the center of the cam 220, and convex portions 220b each of which has the largest radius from the center of the cam 220 are provided on the outer circumference of the cam 220. Furthermore, a transition area is formed on each area from the concave portion 220a to the convex portion 220b shifted anticlockwise in such a shape that the radius of the transition area from the center of the cam 220 smoothly changes. After passing through the convex portion 220b, the radius directly changes to the radius of the concave portion 220a. In this embodiment, four pairs of the concave portion 220a and the convex portion 220b are provided on the outer circumference of the cam 220 at equal intervals.

On the other hand, as illustrated in Fig. 2B, the cartridge 300 includes a reservoir 320 for storing fluid such as a liquid medicine, a tube 330 made of elastic material, an upstream path 340 for guiding the fluid within the reservoir 320 toward the tube 330, a downstream path 342 for guiding the fluid from the tube 330 to an outlet port 350, a shaft holding unit 334 to which a plurality of (seven in this embodiment) pressing shafts 336 for pressing the tube 330 are attached, and others. In this embodiment, the tube 330 is constituted by a silicon tube.

As will be described later in detail, the upstream path 340 and the downstream path 342 are flow paths formed by the first cartridge case 310 and the second cartridge case 312 which constitute the external frame of the cartridge 300. The tube 330 is connected with the upstream path 340 by a junction connector 344, and the tube 330 is connected with the downstream path 342 by a junction connector 346. Fig. 2B shows the structure assuming that the first cartridge case 310 is made of transparent material.

The tube 330 is disposed in a circular-arc shape along a guide wall 332 which regulates the position of the tube 330. The shaft holding unit 334 has the plural pressing shafts 336 radially disposed at equal intervals around the same center as that of the circular-arc shape of the tube 330. The respective pressing shafts 336 are inserted into through holes formed on the shaft holding unit 334 in such a manner that the pressing shafts 336 can reciprocate within the holes. The pressing shafts 336 having this structure shift toward the tube 330 to press the tube 330 from the side opposite to the guide wall 332. However, when the cartridge 300 is removed from the main body 200, the pressing shafts 336 are withdrawn by the elastic force of the tube 330. Thus, the tube 330 is not pressed in this condition.

The tube pump 100 in this embodiment is constituted by the main body 200 and the cartridge 300 having these structures, and functions as a fluid transporter when the cartridge 300 is attached to the main body 200 as one body. The fluid transportation operation performed by the tube pump 100 in this embodiment is hereinafter explained.

### B. Fluid Transportation Operation in This Embodiment

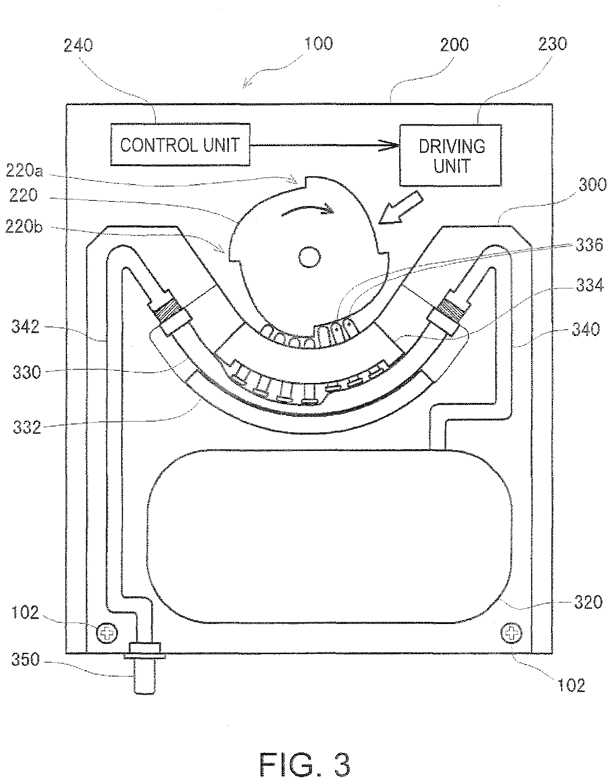

Fig. 3 illustrates a condition in which the cartridge 300 is attached to the main body 200 of the tube pump 100 in this embodiment. The tube pump 100 in this embodiment prevents separation of the cartridge 300 from the main body 200 by using a fixing screw 102 threaded from the first main body case 210 side after attachment of the cartridge 300 to the attachment space 250 of the main body 200.

As illustrated in the figure, under the condition in which the cartridge 300 is attached to the main body 200, one ends (ends on the side opposite to the side contacting the tube 330) of the plural (seven in this embodiment) pressing shafts 336 attached to the shaft holding unit 334 of the cartridge 300 contact the outer circumferential surface of the cam 220 mounted on the main body 200. As explained above, the concave portions 220a, the convex portions 220b, and the transition areas formed on the areas from the concave portions 220a to the convex portions 220b are formed on the outer circumference of the cam 220. The pressing shafts 336 contacting the concave portion 220a are still withdrawn by the elastic force of the tube 330 and thus do not press the tube 330. However, the pressing shafts 336 contacting the transition area and the convex portion 220b are pushed toward the tube 330 and thus press the tube 330 against the guide wall 332. Particularly, the pressing shafts 336 contacting the convex portion 220b press the tube 330 to the largest degree.

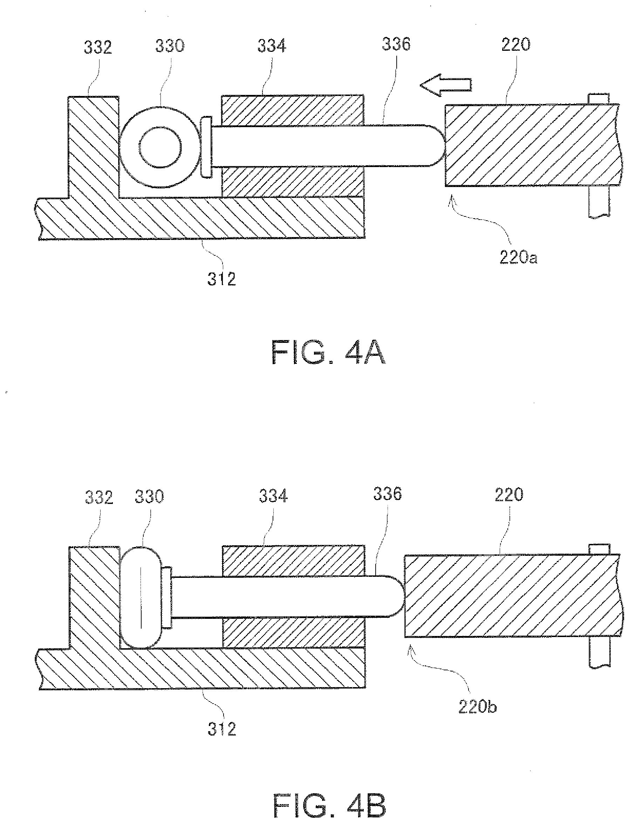

Figs. 4A and 4B are cross-sectional views illustrating the tube 330 pressed by the pressing shaft 336. Fig. 4A shows a condition in which the pressing shaft 336 contacts the concave portion 220a of the cam 220. As explained above, the tube 330 is disposed in the circular-arc shape along the guide wall 332 in this embodiment. The plural pressing shafts 336 inserted into the through holes of the shaft holding unit 334 are radially disposed around the same center as that of the circular-arc shape of the tube 330. The center of the circular-arc shape of the tube 330 agrees with the rotation center of the cam 220 under the condition that the cartridge 300 is attached to the main body 200. Each length of the pressing shafts 336 is determined as the minimum distance between the side surface of the tube 330 and each of the concave portions 220a on the outer circumferential surface of the cam 220. Thus, the pressing shaft 336 contacting the concave portion 220a of the cam 220 does not press the tube 330. In this embodiment, the guide wall 332 is formed integrally with the second cartridge case 312, and the shaft holding unit 334 is fixed to the second cartridge case 312.

When the cam 220 is rotated clockwise from the condition shown in Fig. 4A by the operation of the driving unit 230, the pressing shaft 336 contacting the concave portion 220a of the cam 220 comes into contact with the transition area whose radius from the center of the cam 220 gradually increases. As a result, the pressing shaft 336 is gradually pushed toward the tube 330 to press the tube 330 against the guide wall 332.

With further rotation of the cam 220, the pressing shaft 336 comes to contact the convex portion 220b of the cam 220, where the pressing shaft 336 is pushed toward the tube 330 to the largest degree. In this condition, the tube 330 is pressed by the pressing shaft 336 most greatly against the guide wall 332, and the entire areas of the inner surface of the tube 330 are joined to each other to be completely closed.

Accordingly, the position of the tube 330 pressed by the pressing shaft 336 contacting the convex portion 220b of the cam 220 is completely closed. This closed position of the tube 330 shifts in accordance with the rotation of the cam 220 by the operation of the driving unit 230 (see Fig. 3). Thus, fluid within the tube 330 is sequentially pushed out toward the downstream side. Moreover, the cam 220 has the concave portions 220a each of which is disposed immediately after the corresponding convex portion 220b. Thus, after passing through the convex portion 220b by the rotation of the cam 220, the pressing shaft 336 is pushed and returned by the restoring force of the tube 330 at the closed position of the tube 330, and fluid is sucked from the upstream side. By continuously repeating these actions, the tube pump 100 can transport fluid stored within the reservoir 320 toward the outlet port 350. According to this embodiment, the four convex portions 220b are provided on the cam 220 at equal intervals such that the tube 330 can be kept closed at one point by the press of any of the convex portions 220b. Thus, reverse flow of the fluid can be avoided.

As described above, the tube pump 100 in this embodiment guides the fluid within the reservoir 320 toward the tube 330 through the upstream path 340, and guides the fluid from the tube 330 toward the outlet port 350 through the downstream path 342. The details of the respective structures of the upstream path 340 and the downstream path 342 provided on the cartridge 300, and the connections between the two paths and the tube 330 are now explained.

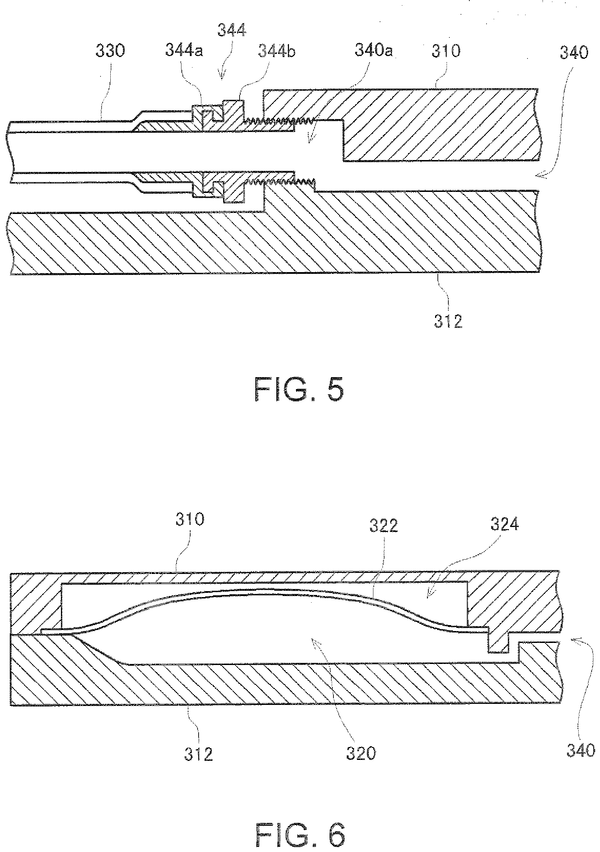

Fig. 5 is a cross-sectional view illustrating the structure of the upstream path 340 provided on the cartridge 300, and the connection between the upstream path 340 and the tube 330 according to this embodiment. As illustrated in the figure, the upstream path 340 in this embodiment is recessed as a groove at least either on the first cartridge case 310 or on the second cartridge case 312, and is formed by affixing the first cartridge case 310 and the second cartridge case 312. The inside diameter of the upstream path 340 is smaller than the inside diameter of the tube 330.

A connection space 340a as a large recessed area on the first cartridge case 310 is formed at the tube 330 side end of the upstream path 340 for connection with the tube 330, and the junction connector 344 is attached to the connection space 340a. The junction connector 344 in this embodiment is a so-called rotary connector which has a first member 344a and a second member 344b connected to each other in such a manner that the members 344a and 344b can rotate relative to each other. The first member 344a is inserted into the upstream side of the tube 330 and fixed thereto. As illustrated in the figure, a male screw is formed on the side surface of the second member 344b, and a corresponding female screw is formed on the inner surface of the connection space 340a. According to this structure, the depth of insertion of the junction connector 344 into the connection space 340a can be easily adjusted by rotating the second member 344b into the connection space 340a. The structure of the downstream path 342, the connection between the downstream path 342 and the tube 330 via the junction connector 346 are basically similar to those associated with the upstream path 340, and the same explanation is not repeated herein.

According to the tube pump 100 in this embodiment, therefore, the length of the tube 330 attached to the cartridge 300 can be easily controlled by the depth of the insertion of the junction connector 344 into the connection space 340a. Thus, the accuracy of fluid transportation increases. In case of a typical tube formed by extrusion molding, the inside diameter of the tube varies for each manufacturing lot, and the flow amount of fluid flowing within the tube changes accordingly. Thus, for accurate transportation of a specified amount of fluid, complicated corrections such as control over the press of the pressing shafts against the tube are required. According to the tube pump 100 in this embodiment, however, the degree of tension (expansion and contraction) of the tube 330 attached to the cartridge 300 can be controlled by varying the depth of insertion of the junction connector 344 into the connection space 340a, and thus the variation of the inside diameter of the tube 330 produced in the manufacture can be easily corrected. For example, when the inside diameter of the tube 330 is larger than the standard diameter due to the variation in the manufacture, the junction connector 344 is inserted into the connection space 340a more deeply than in the standard condition to increase the tension (expansion) of the tube 330. As a result, the inside diameter of the tube 330 becomes smaller, and thus the effect given by the variation of the inside diameter of the tube 330 produced in the manufacture decreases. Accordingly, the accuracy of fluid transportation improves.

The structure of the reservoir 320 formed in the cartridge 300 for storing fluid is now explained. Fig. 6 is a cross-sectional view illustrating the structure of the reservoir 320 included in the cartridge 300 according to this embodiment. As can be seen from the figure, a part of the reservoir 320 in this embodiment (the lower part of the reservoir 320 in Fig. 6) is formed integrally with the second cartridge case 312, and the remaining part (the upper part of the reservoir 320 in Fig. 6) is constituted by a film 322 made of elastic material (silicon material in this embodiment). The film 322 is spread in such a manner as to cover a storage space 324 recessed on the first cartridge case 310, and the reservoir 320 is formed when the first cartridge case 310 and the second cartridge case 312 are affixed to each other. The part of the reservoir 320 formed integrally with the second cartridge case 312 is connected with the upstream path 340 such that the fluid stored in the reservoir 320 can be guided through the upstream path 340 toward the tube 330. Fig. 6 shows a condition in which the reservoir 320 is filled with fluid with the film 322 expanded in a convex shape. When the fluid within the reservoir 320 is transported toward the outlet port 350 by the operation of the tube pump 100, the shape of the film 322 changes from the expanded and convexed shape to a shriveled and concaved shape by the decrease of the fluid within the reservoir 320. According to this embodiment, the second cartridge case 312 has a not-shown septum 326 as a port through which fluid is introduced from the outside into the reservoir 320 such that fluid can be injected into the reservoir 320 via the septum 326.

According to the tube pump 100 in this embodiment, therefore, the cartridge 300 including the tube 330 is so constructed as to be attachable to and detachable from the main body 200 on which the cam 220 is mounted, and the tube 330 is pushed by the cam 220 via the pressing shafts 336 when the cartridge 300 is attached to the main body 200. Thus, lowering of the accuracy of fluid transportation caused by continuous press of the tube 330 can be avoided. When the tube pump 100 has such a structure which cannot separate the cartridge 300 from the main body 200 as a structure different from the structure in this embodiment, one position of the tube 330 is kept closed by continuous press during the period from the manufacture of the tube pump 100 to the practical use. In this case, the pressed position of the tube 330 deteriorates and does not return to the original state after an elapse of long time without use, and thus the accuracy of fluid transportation lowers (the flow amount of fluid decreases). When the cartridge 300 is attachable to and detachable from the main body 200 as in this embodiment, however, the tube 330 is not pressed until the cartridge 300 is attached to the main body 200 for practical use of the tube pump 100. Thus, the tube 330 does not deteriorate, allowing the accuracy of fluid transportation to be kept unchanged from the time of manufacture. Even after attachment of the cartridge 300 to the main body 200 and start of use of the tube pump 100, improvement in the durability of the tube 330 and prevention of decrease in the accuracy of fluid transportation can be both achieved by removing the cartridge 300 from the main body 200 while the tube pump 100 is not being operated.

According to the tube pump 100 in this embodiment, the portion included in the transportation path for fluid which extends from the reservoir 320 to the outlet port 350 and pressed by the cam 220 via the pressing shafts 366 is constituted by the tube 330. On the other hand, the upstream path 340 from the reservoir 320 to the tube 330 and the downstream path 342 from the tube 330 to the outlet port 350 are formed integrally with the case of the cartridge 300 (the first cartridge case 310 and the second cartridge case 312). Thus, the accuracy of fluid transportation can increase. In general, for transporting a liquid medicine by using a tube pump, a silicon tube is employed as the tube of the tube pump in most cases considering chemical resistance and the like. However, the liquid medicine easily evaporates (the vaporized liquid medicine easily goes out) from the silicon tube, and gradually decreases during transportation. In this case, accurate transportation of a specified amount of the liquid medicine is difficult. Moreover, the medicine evaporated and adhering to the driving unit 230, the control unit 240 and other components prevents normal operation and control, which also makes it difficult to transport the liquid medicine with high accuracy. Considering these points, the tube pump 100 in this embodiment limits the length of the tube 330, which is also a silicon tube, to a length only required for receiving press by the plural pressing shafts 336, and forms the upstream path 340 and the downstream path 342 in connection with the tube 330 integrally with the case of the cartridge 300 (the first cartridge case 310 and the second cartridge case 312) by using plastic material. Since the upstream path 340 and the downstream path 342 have a higher gas-barrier property for shielding gas than that of the tube 330 (silicon tube), evaporation of the liquid medicine during transportation can be decreased more than in the structure which forms the entire transportation path from the reservoir 320 to the outlet port 350 by the tube 330. Thus, the accuracy of fluid transportation improves. In view of reduction of the evaporation amount of the liquid medicine from the tube 330, it is preferable that the length of the tube 330 is shorter than the sum of the lengths of the upstream path 340 and the downstream path 342.

Moreover, the structure which limits the length of the tube 330 to the length only required for receiving press by the plural pressing shafts 336 and forms the upstream path 340 and the downstream path 342 in connection with the tube 330 integrally with the case of the cartridge 300 (the first cartridge case 310 and the second cartridge case 312) contributes to the improvement of the accuracy of fluid transportation in the following viewpoints as well. Generally, the inside diameter of the tube 330 varies for each manufacturing lot and greatly affects the accuracy of fluid transportation. However, in case of the upstream path 340 and the downstream path 342 formed integrally with the case of the cartridge 300, the accuracy of the respective dimensions is higher. Therefore, the accuracy of fluid transportation becomes higher than that of the structure which forms the entire transportation path from the reservoir 320 to the outlet port 350 by the tube 330. In case of a typical tube formed by extrusion molding, the inside diameter of the tube varies for each tube. According to the short tube 330 which has only the length required for receiving press by the plural pressing shafts 336 and thus can be shaped by mold forming, however, the variation of the inside diameter can be reduced. Accordingly, the accuracy of fluid transportation can further increase.

Furthermore, since the upstream path 340 and the downstream path 342 connected to the tube 330 are formed integrally with the case of the cartridge 300, the size of the tube pump 100 can be reduced. In case of a structure which draws the tube 330 in a circular shape within the cartridge 300 to form the entire transportation path from the reservoir 320 to the outlet port 350 by the tube 330 as a structure different from the structure in this embodiment, a radius of curvature R needs to be large enough so as not to bend and close the tube 330. However, when the part of the transportation path other than the tube 330 is formed by the upstream path 340 and the downstream path 342 formed integrally with the case of the cartridge 300 with limitation to the length of the tube 330 constituting the transportation path as in this embodiment, the radius of curvature R of each of the upstream path 340 and the downstream path 342 can be reduced (the path can be bended at an acute angle). Thus, the degree of freedom for the layout of the transportation path increases, and the tube pump 100 becomes compact.

In addition, when the length of the tube 330 constituting the transportation path is limited, the attachment error of the tube 330 (expansion of the tube 330 caused by pulling or the like) is less likely to be produced than in the structure which draws the tube 330 in a circular shape from the reservoir 320 to the outlet port 350. Thus, the tube pump 100 can be easily manufactured. Also, when the length of the tube 330 is limited to the length required for receiving press by the plural pressing shafts 336, the amount of the expensive tube used for constituting the transportation path decreases. Thus, the manufacturing cost of the tube pump 100 can be reduced.

According to the tube pump 100 in this embodiment, the inside diameter of each of the upstream path 340 and the downstream path 342 is made smaller than the inside diameter of the tube 330 for the following reason. At the time of the manufacture, the transportation path of the cartridge 300 from the reservoir 320 to the outlet port 350 is not filled with fluid. When the cartridge 300 is attached to the main body 200 for using the tube pump 200 (for delivering fluid through the outlet port 350), initial filling for filling the transportation path with fluid needs to be completed in the first place. In this case, the flow amount of the fluid flowing within the transportation path is determined by the inside diameter of the pressed tube 330 and the rotation speed of the cam 220. Also, each flow speed of the fluid in the upstream path 340 and in the downstream path 342 increases as each inside diameter of the upstream path 340 and the downstream path 342 decreases. Therefore, when each inside diameter of the upstream path 340 and the downstream path 342 is made smaller than the inside diameter of the tube 330, the time required for the initial filling becomes shorter than that time required in the structure which forms the entire transportation path by the tube 330. When the tube 330 is narrowed (the inside diameter of the tube 330 is reduced), the effect of evaporation of the liquid medicine from the tube 330 becomes remarkable. However, when each inside diameter of the upstream path 340 and the downstream path 342 formed integrally with the case of the cartridge 300 is decreased, this problem can be avoided.

Furthermore, according to the tube pump 100 in this embodiment, the structure of the reservoir 320 is also characterized by the upper part of the reservoir 320 constituted by the silicon film 322 and the lower part of the reservoir 320 formed integrally with the second cartridge case 312. When the reservoir 320 is formed by the film 322 made of elastic material (such as silicon), the shape of the film 322 changes in accordance with decrease in the fluid within the reservoir 320 and thus reduces the volume of the reservoir 320. In this case, the pressure within the reservoir 320 does not become negative pressure, and thus the outside air and the like do not enter the reservoir 320. This condition is preferable for the reasons that the whole fluid within the reservoir 320 can be transported with no part left, and that mixture of bubbles into the liquid medicine can be reduced when the fluid to be transported is a liquid medicine. However, as explained above, the silicon film 322 has excellent chemical resistance and flexibility but allows a liquid medicine to be easily evaporated from the silicon film 322. Thus, when the entire reservoir 320 is formed by the silicon film 322, the loss of the evaporated liquid medicine increases. In case of the structure which forms the upper part of the reservoir 320 by the silicon film 322 and forms the lower part of the reservoir 320 integrally with the second cartridge case 312 as in this embodiment, however, the area from which the liquid medicine evaporates becomes smaller than that area in the structure which forms the entire reservoir 320 by the silicon film 322. Thus, the loss of the evaporated liquid medicine decreases.

While the fluid transporter according to the embodiment of the invention has been described, the invention is not limited to all the points of the embodiment shown herein but may be practiced otherwise without departing from the scope of the invention, as defined by the claims.

For example, while the tube 330 is pressed by the plural pressing shafts 336 in the embodiment described herein, the tube 330 may be directly pressed by the cam 220. However, as in the embodiment, when the tube 330 is pressed not directly by the cam 220 but indirectly via the pressing shafts 336, the cam 220 does not slide on the side surface of the tube 330. In this case, abrasion of the tube 330 decreases, and thus durability of the tube 330 increases.

## Claims

1. A fluid transporter which presses a tube (330) made of elastic material by using a pressing member (336) to close at least one position of the tube, and shifts the closed position to transport fluid within the tube (330), comprising:
a main body case (200) on which a driving unit (230) for driving the pressing member to shift the closed position is provided; and
an attachment case (300) which is so constructed as to be attachable to and detachable from the main body case (200) and has a flow path (340, 342) through which the fluid to be transported passes,
wherein
the portion of the flow path (340, 342) pressed by the pressing member is formed by the tube (330), and
the remaining portion of the flow path is formed integrally with the attachment case,
**characterized by**
a control unit, which has a rotation mechanism and which changes the inside diameter of the tube (330) with the tube attached to the attachment case by expanding and contracting the tube in accordance with rotation of the rotation mechanism,
wherein a connection space (340a) is formed at a tube side end of an upstream path (340) and of a downstream path (342) of the attachment case (300) for connection with the tube (330), and a junction connector (344, 346) fixed to the tube (330) and implemented as a rotary connector is attached to the connection space (340a),
wherein the junction connector (344) comprises a first member (344a) and a second member (344b) connected to each other in such a manner that the members (344a, 344b) can rotate relative to each other, wherein the first member (344a) is inserted into the upstream side of the tube (330) and fixed thereto.

2. The fluid transporter according to claim 1, wherein the pressing member includes a cam (220) provided on the main body case (200), and a plurality of pressing shafts (336) provided on the attachment case to sequentially press the tube by rotation of the cam (220).

3. The fluid transporter according to claim 1 or 2, wherein the proportion of the length of the tube (330) to the entire length of the flow path (340, 342) is set at half or shorter than half the entire length of the flow path.

4. The fluid transporter according to claim 1, 2 or 3, wherein the inside diameter of the portion of the flow path (340, 342) provided on the attachment case is made smaller than the inside diameter of the tube (330).

## Patentansprüche

1. Fluidbeförderer, der einen Schlauch (330) aus elastischem Material durch Anwenden eines Presselements (336) zum Verschließen mindestens einer Position des Schlauchs mit Druck beaufschlagt und die geschlossene Position verschiebt, um Fluid in dem Schlauch (330) zu befördern, umfassend:
ein Hauptkörpergehäuse (200), auf dem eine Antriebseinheit (230) zum Antreiben des Presselements zum Verschieben der geschlossenen Position vorgesehen ist; und
ein Befestigungsgehäuse (300), das so aufgebaut ist, dass es an dem Hauptkörpergehäuse (200) anbringbar und davon lösbar ist und einen Strömungspfad (340, 342) aufweist, durch den das zu befördernde Fluid gelangt,
wobei
der Bereich des mit Druck beaufschlagten Strömungspfads (340, 342) durch das Presselement von dem Schlauch (330) ausgebildet ist, und
der verbleibende Bereich des Strömungspfads einstückig mit dem Befestigungsgehäuse ausgebildet ist,
**gekennzeichnet durch**
eine Steuerungseinheit, die einen Rotationsmechanismus aufweist und die den Innendurchmesser des Schlauchs (330) durch Ausdehnen und Zusammenziehen des Schlauchs gemäß der Rotation des Rotationsmechanismus ändert, wobei der Schlauch an dem Befestigungsgehäuse befestigt ist,
wobei ein Verbindungsraum (340a) an einem Schlauchseitenende eines Stromaufwärtspfads (340) und eines Stromabwärtspfads (342) des Befestigungsgehäuses (300) zur Verbindung mit dem Schlauch (330) ausgebildet ist und ein Zusammenführungsverbinder (344, 346), befestigt an dem Schlauch (330) und als ein Rotationsverbinder eingebaut, an dem Verbindungsraum (340a) befestigt ist,
wobei der Zusammenführungsverbinder (344) ein erstes Element (344a) und ein zweites Element (344b) in einer derartigen Weise miteinander verbunden umfasst, dass die Elemente (344a, 344b) relativ zueinander rotieren können, wobei das erste Element (344a) in die Stromaufwärtsseite des Schlauchs (330) eingeschoben und daran befestigt ist.

2. Fluidbeförderer nach Anspruch 1, wobei das Presselement einen Nocken (220), vorgesehen auf dem Hauptkörpergehäuse (200), und eine Vielzahl von Andruckwellen (336), vorgesehen auf dem Befestigungsgehäuse, zum anschließenden Beaufschlagen des Schlauchs mit Druck durch Rotation des Nockens (220) einschließt.

3. Fluidbeförderer nach Anspruch 1 oder 2, wobei das Verhältnis der Länge des Schlauchs (330) zu der Gesamtlänge des Strömungspfads (340, 342) bei einer Hälfte oder kürzer als die Hälfte der Gesamtlänge des Strömungspfads eingestellt ist.

4. Fluidbeförderer nach Anspruch 1, 2 oder 3, wobei der Innendurchmesser des Bereichs des Strömungspfads (340, 342), vorgesehen auf dem Befestigungsgehäuse, kleiner als der Innendurchmesser des Schlauchs (330) ausgebildet ist.

## Revendications

1. Transporteur de fluide qui presse un tube (330) fait de matériau élastique en utilisant un membre pressant (336) pour fermer au moins une position du tube, et déplace la position fermée pour transporter du fluide à l'intérieur du tube (330), comprenant :
un boîtier de corps principal (200) sur lequel une unité d'entraînement (230) pour entraîner le membre pressant à déplacer la position fermée est fournie ; et
un boîtier d'attachement (300) qui est construit de manière à pouvoir être attaché et détaché du boîtier de corps principal (200) et a un chemin d'écoulement (340, 342) à travers duquel le fluide devant être transporté passe,
dans lequel
la portion du chemin d'écoulement (340, 342) pressée par le membre pressant est formée par le tube (330), et
la portion restante du chemin d'écoulement est formée intégralement avec le boîtier d'attachement,
**caractérisé par**
une unité de contrôle, qui a un mécanisme de rotation et qui change le diamètre intérieur du tube (330) avec le tube attaché au boîtier d'attachement en élargissant et en contractant le tube selon la rotation du mécanisme de rotation,
dans laquelle un espace de connexion (340a) est formé au niveau d'une extrémité côté tube d'un chemin en amont (340) et d'un chemin en aval (342) du boîtier d'attachement (300) pour une connexion avec le tube (330), et un connecteur de jonction (344, 346) fixé au tube (330) et mis en oeuvre sous la forme d'un connecteur rotatif est attaché à l'espace de connexion (340a),
dans lequel le connecteur de jonction (344) comprend un premier membre (344a) et un deuxième membre (344b) connectés l'un à l'autre de telle manière que les membres (344a, 344b) peuvent tourner l'un par rapport à l'autre, dans lequel le premier membre (344a) est inséré à l'intérieur du côté en amont du tube (330) et y est fixé.

2. Transporteur de fluide selon la revendication 1, dans lequel le membre pressant inclut une came (220) fournie sur le boîtier de corps principal (200), et une pluralité d'arbres pressants (336) fournis sur le boîtier d'attachement pour presser de manière séquentielle le tube par la rotation de la came (220).

3. Transporteur de fluide selon la revendication 1 ou 2, dans lequel la proportion de la longueur du tube (330) sur toute la longueur du chemin d'écoulement (340, 342) est réglée à la moitié ou moins de la moitié de toute la longueur du chemin d'écoulement.

4. Transporteur de fluide selon la revendication 1, 2 ou 3, dans lequel le diamètre intérieur de la portion du chemin d'écoulement (340, 342) fournie sur le boîtier d'attachement est réalisé plus petit que le diamètre intérieur du tube (330).
